# FASCICULE DE BREVET EUROPEEN

(11) **EP 3 659 629 B1**
(45) Date de publication et mention de la délivrance du brevet: **07.06.2023**
(21) Numéro de dépôt: 19212877.5
(22) Date de dépôt: 02.12.2019
(51) Int. Cl.: A61L 9/12, B05B 9/00, F16K 1/00

(54) **DIFFUSEUR D'HUILE ESSENTIELLE**
DIFFUSOR VON ÄTHERISCHEN ÖLEN
ESSENTIAL OILS DIFFUSER

(30) Priorité: 30.11.2018 FR 1872159
(43) Date de publication de la demande: 03.06.2020
(73) Titulaire: LASER, 92300 Levallois Perret (FR)
(72) Inventeur: DE POMMEREAU, Ségolène, 75116 PARIS (FR)
(74) Mandataire: Plasseraud IP

(56) Documents cités:
- FR-A1- 2 961 698
- US-A1- 2008 290 120
- US-A1- 2010 243 754

## Description

### Domaine technique

La présente demande concerne le domaine technique des diffuseurs d'huile essentielle et plus particulièrement des diffuseurs électriques d'huile essentielle.

### Technique antérieure

Il est connu aujourd'hui différents types de diffuseurs électriques d'huile essentielle et notamment des diffuseurs avec un disperseur consistant en une pièce piézoélectrique.

Parmi les diffuseurs une distinction est à faire également entre les diffuseurs présentant un réservoir ouvert d'huile essentielle (mélangée ou non à de l'eau) rempli manuellement par l'utilisateur et les diffuseurs avec logement de réception d'un flacon d'huile essentielle. L'avantage de ces derniers est de ne pas nécessiter de remplissage manuel entre chaque utilisation.

De manière générale, ce dernier type de diffuseurs comprend un boitier sur lequel sont disposés une fiche mâle ou un connecteur USB et le logement de réception du flacon ce qui les rend compacts et pratiques à utiliser.

Un flacon d'huile essentielle est dans la grande majorité des cas muni d'un compte-gouttes. Toutefois, pour les diffuseurs avec logement de réception, le compte-gouttes doit généralement être retiré pour permettre d'insérer à l'intérieur du flacon un élément absorbant faisant remonter l'huile par capillarité. Le fait de devoir retirer le compte-gouttes présente l'inconvénient de salir les mains de l'utilisateur.

Dans d'autres cas, le flacon d'huile essentielle est disposé à l'envers pour permettre à l'huile essentielle de s'écouler par gravitation dans un réservoir. Lors du retrait du flacon du logement de réception, il est nécessaire de vider le réservoir du diffuseur à nouveau dans le flacon au risque de très souvent renverser de l'huile essentielle, par exemple sur les mains ou sur un support ce qui oblige un nettoyage des mains et/ou du support et selon l'huile essentielle utilisée, le nettoyage doit être rapidement effectué pour éviter d'abimer les mains et/ou d'endommager le support.

D'autres documents décrivant l'arrière-plan technologique sont : FR2961698 A1, US 2008/290120 A1, et US 2010/243754 A1.

### Exposé de l'invention

Ainsi, l'un des objectifs de la présente invention est de remédier à l'un au moins des inconvénients de l'état de la technique.

Pour cela, est proposé un diffuseur électrique d'huile essentielle selon la revendication 1.

D'autres caractéristiques optionnelles et non limitatives sont décrites dans les revendications dépendantes. Elles peuvent être mises en oeuvre indépendamment les unes des autres ou en combinaison les unes avec les autres.

Le coupe-gouttes peut comprendre :
- un clapet présentant deux positions, une position fermée correspondant à la position fermée du coupe-gouttes et une position ouverte correspondant à la position ouverte du coupe-gouttes ;
- un actionneur pour déplacer le clapet de la position fermée à la position ouverte ;
- un rappel pour déplacer le clapet de la position ouverte à la position fermée ;
la bague de fixation et le coupe-gouttes étant configurés de manière à ce que lors de la fixation du col du flacon à la bague de fixation, celui-ci force l'actionneur à déplacer le clapet de la position fermée à la position ouverte et que lors du retrait du col du flacon de la bague de fixation, le rappel ramène le clapet à sa position fermée.

Le coupe-gouttes peut être formé de :
- une pièce mobile formée d'une base mobile de laquelle s'étend substantiellement perpendiculairement à celle-ci un ou plusieurs doigts d'actionnement formant l'actionneur ;
- une base circulaire comprenant un ou plusieurs premiers orifices, le nombre de premiers orifices étant égal au nombre de doigts d'actionnement, chaque doigt traversant un orifice en position fermée du clapet ;
la base circulaire comprenant un deuxième orifice disposé en face de la base mobile de manière à être obstruable par celle-ci formant ainsi le clapet.

La base circulaire peut être prolongée à sa périphérie d'une jupe s'étendant substantiellement perpendiculairement à la base circulaire, la jupe et la base circulaire formant logement pour la pièce mobile et le rappel.

Le rappel peut être un ressort disposé de manière à forcer la base mobile contre la base circulaire obstruant ainsi le deuxième orifice.

Le diffuseur peut comprendre en outre un logement de ressort disposé sous la pièce mobile.

Le logement de ressort peut être formé d'au moins trois bras coplanaires s'étendant à partir d'un centre et d'autant de doigts de fixation, chacun des doigts de fixation prolongeant l'un des trois bras coplanaires perpendiculairement au plan commun des bras coplanaires ;
et la jupe peut être pourvue d'autant d'orifices que de doigts de fixation et recevant chacun un des doigts de fixation pour la fixation du logement de ressort à la jupe.

Le diffuseur peut comprendre en outre un connecteur à une source d'alimentation. Le connecteur à une source d'alimentation peut être une fiche mâle ou un connecteur USB. Alternativement ou en complément, le diffuseur peut comprendre un module photovoltaïque pour l'alimentation électrique du diffuseur.

Le disperseur peut être un nébuliseur ou une pastille piézoélectrique.

### Brève description des dessins

D'autres caractéristiques, détails et avantages de l'invention apparaitront à la lecture de la description détaillée ci-après, et à l'analyse des dessins annexés, sur lesquels :
**Fig. 1**

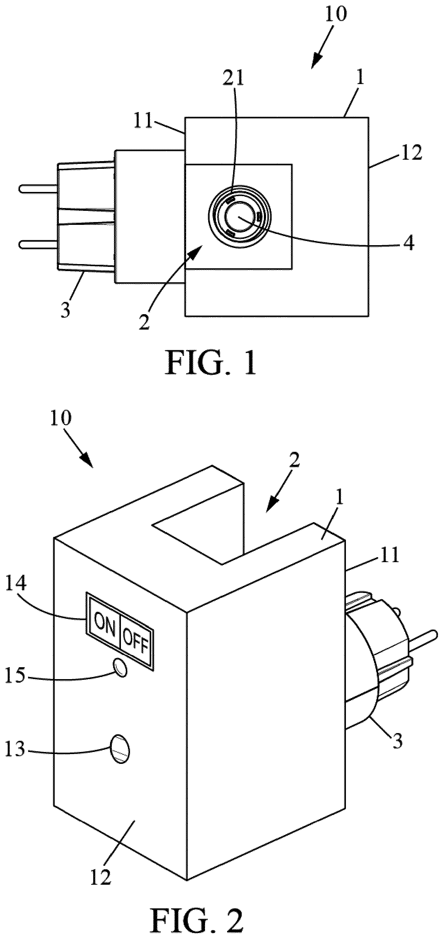

   [Fig. 1] est une vue de dessus d'un diffuseur selon l'invention sans flacon d'huile essentielle ;
**Fig. 2**
   [Fig. 2] est une vue de trois-quarts de l'avant du diffuseur de la figure 1 ;
**Fig. 3**

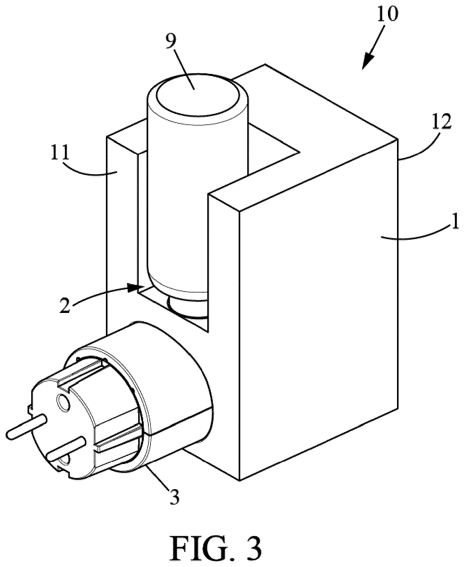

   [Fig. 3] est une vue de trois-quarts de l'arrière du diffuseur de la figure 1 avec un flacon d'huile essentielle ;
**Fig. 4**

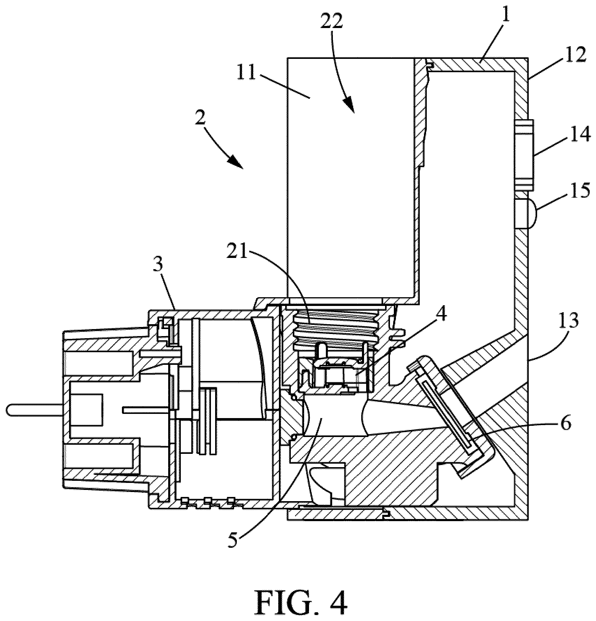

   [Fig. 4] est une vue en coupe du diffuseur de la figure 1 montrant le chemin pris par l'huile essentielle du logement de réception à la sortie de diffusion, le rappel n'étant pas représenté ;
**Fig. 5**

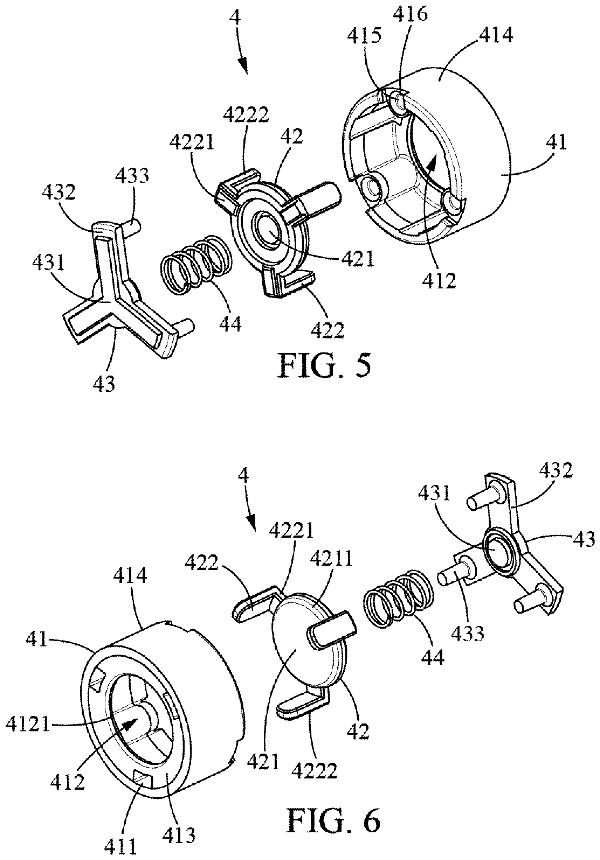

   [Fig. 5] est une vue éclatée d'un coupe-gouttes utilisé dans le diffuseur de la figure 1 regardée dans le sens logement de ressort vers base circulaire ;
**Fig. 6**
   [Fig. 6] est une vue éclatée d'un coupe-gouttes utilisé dans le diffuseur de la figure 1 regardée dans le sens base circulaire vers logement de ressort ; et
**Fig. 7**

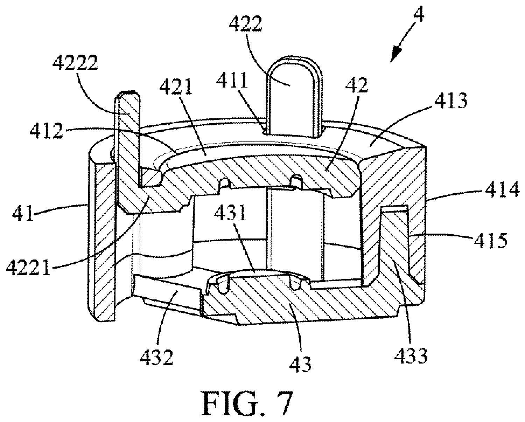

   [Fig. 7] est une vue partiellement en coupe d'un coupe-gouttes utilisé dans le diffuseur de la figure 1, le rappel n'étant pas montré.

### Présentation détaillée de l'invention

Un diffuseur **10** d'huile essentielle selon la présente invention est décrit ci-après en référence aux figures 1 à 7. Dans la suite, les orientations spatiales sont données par rapport à la position normale d'utilisation du diffuseur **10.**

Le diffuseur **10** d'huile essentielle comprend un boitier **1,** un logement de réception **2** d'un flacon **9** d'huile essentielle, un connecteur **3** à une source d'alimentation, un réservoir **5** et un coupe-gouttes **4.** Le logement de réception **2** et le connecteur **3** sont prévus sur le boitier **1** et le coupe-gouttes **4** dans le logement de réception **2** ou juste en dessous de celui-ci. Le logement de réception **2** comprend une bague de fixation **21** du flacon **9.**

Le coupe-gouttes **4** présente une position fermée et une position ouverte et est agencé par rapport à la bague de fixation **21** de manière à ce que le coupe-gouttes **4** soit :
- dans sa position ouverte lorsqu'un flacon **9** est reçu dans le logement de réception **2** et fixé à la bague de fixation **21** reliant fluidiquement le logement de réception **2** et le réservoir **5** ; et
- dans sa position fermée sinon.

Le coupe-gouttes **4** peut comprendre un clapet, un actionneur pour déplacer le clapet et ainsi commander le clapet d'une position fermée correspondant à la position fermée du coupe-gouttes **4** à une position ouverte correspondant à la position ouverte du coupe-gouttes **4,** et un rappel pour déplacer le clapet de la position ouverte à la position fermée.

Auquel cas, la bague de fixation **21** et le coupe-gouttes **4** sont configurés de manière à ce que lors de la fixation du col du flacon **9** à la bague de fixation **21,** le col force l'actionneur à déplacer le clapet de la position fermée à la position ouverte et que lors du retrait du col du flacon **9** de la bague de fixation **21,** le rappel ramène le clapet à sa position fermée.

Plus particulièrement, le coupe-gouttes **4** peut être formé d'une pièce mobile **42,** et une base circulaire **41.**

La pièce mobile **42** comprend une base mobile **421** de laquelle s'étendent substantiellement perpendiculairement à celle-ci un ou plusieurs doigts d'actionnement **422** formant l'actionneur, éventuellement après une prolongation coplanaire **4221** à la base mobile **421.** De préférence, la base mobile **421** présente une forme discoïde. Chacun des doigts d'actionnement **422** présente une première partie de prolongement **4221** colinéaire à la base mobile **421** et une partie saillante **4222** s'étendant perpendiculairement à la surface discoïde de la base mobile **421.** De préférence, la largeur de la partie de prolongement **4221** et celle de la partie saillante **4222** sont égales. La partie saillante **4222** présente de préférence une extrémité libre arrondie. La surface des parties de prolongement **4221** du même côté que la direction d'extension des parties saillantes **4222** est en retrait par rapport à la surface de la base mobile **421** du même côté. De préférence, chaque doigt d'actionnement **422** s'étend radialement à partir de la base mobile **421** discoïde. S'il y a une pluralité de doigts d'actionnement **422,** ils sont de préférence répartis angulairement de manière homogène autour de la base mobile **421.**

La base circulaire **41** comprend un ou plusieurs premiers orifices **411,** le nombre de premiers orifices **411** étant égal au nombre de doigts d'actionnement **422,** chaque doigt traversant un premier orifice **411** en position fermée du clapet.

La base circulaire **41** comprend encore un deuxième orifice **412** disposé en face de la base mobile **421** de manière à être obstruable par celle-ci et formant ainsi le clapet.

De préférence, la taille de la base mobile **421** est légèrement plus grande que la taille du deuxième orifice **412.** De préférence, la base mobile **421** et le deuxième orifice **412** présente une même géométrie. De préférence, la base circulaire **41** présente une forme annulaire circulaire avec le deuxième orifice circulaire **412** au centre et les premiers orifices **411** placés sur l'anneau **413** et ayant la même répartition angulaire que les doigts d'actionnement **422.** De préférence, la base circulaire **41** présente un chanfrein **4121** sur son bord intérieur entourant le deuxième orifice **412** et la base mobile **421** présente également un chanfrein **4211** sur son bord extérieur facilitant ainsi l'ajustement de l'un à l'autre pour former la position fermée du coupe-gouttes **4.**

De préférence, la taille et la forme des premiers orifices **411** sont adaptées à celle des doigts d'actionnement **422** et notamment de leurs parties saillantes **4222** pour un déplacement par translation des parties saillantes **4222** au travers des premiers orifices **411** sans jeu. Les premiers orifices **411** peuvent également servir de guide pour le déplacement des doigts d'actionnement **422.**

La base circulaire **41** peut être prolongée à sa périphérie d'une jupe **414** s'étendant substantiellement perpendiculairement à la base circulaire **41,** la jupe **414** et la base circulaire **41** formant logement pour la pièce mobile **42** et le rappel **44.** La jupe **414** est de préférence cylindrique, toujours de préférence à base circulaire **41.**

Le rappel **44** est de préférence un ressort disposé de manière à forcer la base mobile **421** contre la base circulaire **41** obstruant ainsi le deuxième orifice **412.** Le ressort **44** est de préférence un ressort en hélice cylindrique disposé centralement par rapport à la base mobile **421.** Bien entendu le rappel **44** peut être tout moyen permettant au clapet de revenir dans sa position fermée.

Le diffuseur **10** peut comprendre en outre un logement de ressort **43** disposé sous la pièce mobile **42.** Le logement de ressort **43** sert notamment de butée fixe pour le ressort **44.** Par exemple, le logement de ressort **43** est formé d'au moins trois bras coplanaires **432** s'étendant à partir d'un centre **431** (de préférence répartis angulairement de manière régulière) et d'autant de doigts de fixation **433,** chacun des doigts de fixation **433** prolongeant l'un des trois bras coplanaires **432** perpendiculairement au plan commun des bras coplanaires **432.** Auquel cas, la jupe **414** est avantageusement pourvue d'autant d'orifices de fixation **415** qu'il y a de doigts de fixation **433.** Chacune des orifices de fixation **415** reçoit un des doigts de fixation **433** pour la fixation du logement de ressort **43** à la jupe **414.** De préférence encore, la jupe **414** peut comprendre autant d'encoches **416** que de doigts de fixation **433** du logement de ressort **43** pour leur indexation. Les encoches **416** sont réalisées notamment sur le bord circulaire libre de la jupe **414.**

La bague de fixation **21** du logement de réception **2** présente un taraudage pour la fixation par vissage du col du flacon **9** d'huile essentielle. La hauteur de la bague de fixation **21** peut correspondre à la hauteur du col du flacon **9** ou plus petite. La partie filetée du col présente généralement une hauteur comprise entre 8 et 15 mm, plus généralement entre 11 et 12 mm. Par exemple la hauteur de la bague de fixation **21** est comprise entre 8 et 15 mm, préférentiellement entre 11 et 12 mm.

La bague de fixation **21** est disposée juste au-dessus du coupe-gouttes **4,** notamment de manière à ce que les doigts d'actionnement **422** s'étendent au moins partiellement dans la bague de fixation **21** de sorte à être actionnable par le col du flacon **9.**

Le logement de réception **2** peut aussi comprendre une chambre ouverte **22** dans laquelle le corps du flacon **9** est logeable. La chambre ouverte **22** est disposée au-dessus de la bague de fixation **21.** Ainsi, lors de l'utilisation normale du diffuseur **10,** le flacon **9** est maintenu à l'envers pour que l'huile puisse s'écouler hors de celui-ci dans le diffuseur **10** par gravité. La taille de la chambre ouverte **22** est choisie de manière à pouvoir y loger au moins un flacon **9** de 5 mL, de préférence jusqu'à 10 mL, jusqu'à 15 mL, jusqu'à 30 mL. Cette chambre ouverte **22** est de préférence réalisée dans le boitier **1.**

Le réservoir **5** est disposé de préférence sous le coupe-gouttes **4.** La capacité du réservoir **5** est de préférence inférieure à 5 mL, de préférence inférieure à 3 mL, toujours de préférence entre 1 mL et 2 mL. Mais de manière générale, sa capacité n'a pas d'importance, il suffit qu'elle soit suffisante pour permettre la diffusion de l'huile essentielle selon le disperseur choisi. En effet, le fait que le flacon **9** d'huile essentielle reste fixé au diffuseur **10** assure l'alimentation du réservoir **5** en huile jusqu'à ce que le flacon **9** soit vide ou presque vide (le compte-gouttes ne permettant pas toujours de vider entièrement le flacon **9**).

Le réservoir **5** est relié fluidiquement à une sortie de diffusion **13** réalisée dans le boitier **1.** Cette sortie de diffusion **13** permet à l'huile de sortir du diffuseur **10** et de se répandre dans la pièce où est placé celui-ci.

Le disperseur **6** peut être un nébuliseur ou une pastille piézoélectrique. Il est disposé dans le réservoir **5.**

Le connecteur **3** à une source d'alimentation peut être une fiche mâle ou un connecteur USB. Dans le cas d'une fiche mâle, celle-ci est de préférence symétrique dans le plan formé par la borne de phase et la borne neutre afin de permettre la bonne position du diffuseur **10** selon l'orientation des prises femelles. Alternativement ou en complément, le diffuseur **10** d'huile essentielle peut comprendre un module photovoltaïque (mini panneau solaire) permettant l'alimentation électrique du diffuseur par énergie solaire. Lorsque le diffuseur **10** comprend à la fois le connecteur **3** et le module photovoltaïque, cela permet à la fois d'utiliser l'énergie solaire lorsque celle-ci est disponible ou une autre source d'alimentation lorsque l'énergie solaire n'est pas disponible par exemple par temps couvert, par temps pluvieux ou la nuit.

De préférence, le connecteur **3** et le logement de réception **2** sont disposés du même côté du boitier **1,** appelé l'arrière du boitier **11.** Ainsi, l'avant du boitier **12** peut être utilisé pour cacher en partie le flacon **9** et la fiche et également pour porter un élément de décoration.

Le diffuseur **10** comprend comme tout diffuseur électrique une électronique (non illustrée sur les figures) pour la commande de la diffusion de l'huile essentielle. Cette électronique est disposée dans le boitier **1** et connecté électriquement au connecteur **3** d'alimentation.

Le diffuseur **10** peut comprendre en outre un interrupteur **14** comprenant au moins deux positions : marche et arrêt. Alternativement, l'interrupteur **14** comprend plus de deux positions dont l'une pour l'arrêt et deux pour deux modes de fonctionnement différents du diffuseur **10.** De préférence, l'interrupteur **14** est un bouton poussoir réalisé sur le boitier **1,** avantageusement à l'avant du boitier **12.**

Le diffuseur **10** peut encore comprendre un indicateur **15** de fonctionnement pour indiquer à l'utilisateur qu'il est en marche. Cet indicateur **15** est de préférence une diode électroluminescente qui est allumée quand le diffuseur **10** est en fonctionnement. L'indicateur **15** est de préférence disposé sur le boitier **1,** avantageusement sur la partie avant du boitier **1** de sorte à demeurer visible quand le diffuseur **10** est branché par le connecteur **3** à une source d'alimentation. L'indicateur **15** peut en outre comprendre un diffuseur optique couplé à la DEL, la DEL éclairant le diffuseur optique et ce dernier propageant la lumière émise par la DEL de sorte que ce qui est visible par l'utilisateur ait une plus grande taille.

Avantageusement, l'indicateur **15** peut être combiné à l'interrupteur.

Le mode de fonctionnement est ci-après décrit en référence à un diffuseur **10** comprenant l'ensemble des caractéristiques décrites ci-dessus. Dans les modes de réalisation sans l'une ou l'autre des caractéristiques, la description du fonctionnement y correspondant peut être simplement ignorée.

Le coupe-gouttes **4** est par défaut en position fermée, notamment le rappel **44** force le clapet dans sa position fermée, par exemple le ressort **44** exerce une force contre la base mobile **421** qui vient alors obstruer le deuxième orifice **412** de la base circulaire **41.** Le déplacement de la base mobile **421** est limité par la base circulaire **41** elle-même, par exemple par la partie annulaire **413** de celle-ci. Dans cette position, les doigts d'actionnement **422** font saillie complètement hors des premiers orifices **411.**

Un flacon **9** d'huile essentielle est ensuite fixé au diffuseur **10** par le logement de réception **2,** par exemple en vissant le col du flacon **9** à la bague de fixation **21** du logement de réception **2.**

Lors de la fixation du flacon **9** au logement de réception **2,** le col du flacon **9** entre en contact avec l'actionneur et force l'actionneur à entrainer le clapet dans sa position ouverte, par exemple en repoussant les doigts d'actionnement **422** qui transmettent la force exercée par le col du flacon **9** à la base mobile **421** ouvrant ainsi le clapet.

L'huile essentielle peut alors s'écouler du flacon **9,** toujours doté de son compte-gouttes, vers le réservoir **5.**

Le diffuseur **10** est ensuite connecté à une source d'alimentation via le connecteur 3, par exemple en enfonçant la fiche mâle à une prise secteur. Il convient de noter que cette étape peut être effectuée avant la fixation du flacon **9,** bien que la réaliser après la fixation rend la fixation plus aisée.

Dans le cas où le diffuseur **10** ne comprend pas d'interrupteur, le diffuseur **10** commence à fonctionner dès qu'il est branché. Dans le cas où le diffuseur **10** dispose d'un interrupteur **14,** il ne commence à fonctionne qu'à partir du moment où l'utilisateur met l'interrupteur **14** en position marche.

L'huile essentielle est ensuite diffusée grâce au disperseur **6.** Le disperseur **6** nébulise ou brume l'huile essentielle qui ressort par la sortie de diffusion **13.**

Pendant le fonctionnement du diffuseur **10** et si celui-ci dispose d'un indicateur **15** celui-ci est actionné, par exemple la DEL est allumée. L'utilisateur peut alors savoir que le diffuseur **10** est en fonctionnement en voyant la DEL ou le diffuseur optique.

Pour arrêter l'appareil, soit l'utilisateur débranche le diffuseur **10** et/ou met l'interrupteur **14** en position arrêt selon le cas applicable. Le disperseur **6** cesse alors de diffuser l'huile essentielle. L'utilisateur peut alors retirer le flacon **9.** Ce faisant, le rappel **44** ramène le clapet dans sa position fermée, par exemple le ressort **44** repousse la base mobile **421** contre la base circulaire **41.** L'huile essentielle contenue dans le réservoir **5** ne peut donc pas en sortir par le logement de réception **2** ce qui évite de se salir les mains et/ou un support en manipulant le diffuseur **10** et le flacon **9.** Par ailleurs, il n'y a pas besoin de vider le réservoir **5** du diffuseur **10** dans le flacon **9.**

Dans le cas où il reste de l'huile essentielle dans le réservoir **5** du diffuseur **10,** il est possible de faire fonctionner le diffuseur **10** et avoir une diffusion d'huile essentielle même en l'absence du flacon **9,** c'est-à-dire quand le clapet est en position fermée.

## Revendications

1. Diffuseur (10) électrique d'huile essentielle comprenant :
- un boitier (1) ;
- un logement de réception (2) d'un flacon d'huile essentielle prévu sur le boitier et muni d'une bague de fixation (21) ;
- un réservoir (5) muni d'un disperseur (6) d'huile essentielle ;
**caractérisé en ce que** le diffuseur comprend en outre un coupe-gouttes (4) dans le logement de réception ou juste en dessous de celui-ci et présentant une position fermée et une position ouverte ;
la bague de fixation et le coupe-gouttes étant agencés de manière à ce que le coupe-gouttes soit :
- dans sa position ouverte lorsqu'un flacon est reçu dans le logement de réception et fixé à la bague de fixation reliant fluidiquement le logement de réception et le réservoir ; et
- dans sa position fermée sinon ;
dans lequel la bague de fixation présente un taraudage pour la fixation par vissage du col du flacon d'huile essentielle ;
dans lequel le coupe-gouttes est logé sous la bague de fixation.

2. Diffuseur selon la revendication 1, dans lequel le coupe-gouttes comprend :
- un clapet (412, 421) présentant deux positions, une position fermée correspondant à la position fermée du coupe-gouttes et une position ouverte correspondant à la position ouverte du coupe-gouttes ;
- un actionneur (422) pour déplacer le clapet de la position fermée à la position ouverte ;
- un rappel (44) pour déplacer le clapet de la position ouverte à la position fermée ;
la bague de fixation et le coupe-gouttes étant configurés de manière à ce que lors de la fixation du col du flacon à la bague de fixation, celui-ci force l'actionneur à déplacer le clapet de la position fermée à la position ouverte et que lors du retrait du col du flacon de la bague de fixation, le rappel ramène le clapet à sa position fermée.

3. Diffuseur selon la revendication 2, dans lequel le coupe-gouttes est formé de :
- une pièce mobile (42) formée d'une base mobile (421) de laquelle s'étend substantiellement perpendiculairement à celle-ci un ou plusieurs doigts d'actionnement (422) formant l'actionneur ;
- une base circulaire (41) comprenant un ou plusieurs premiers orifices (411), le nombre de premiers orifices étant égal au nombre de doigts d'actionnement, chaque doigt traversant un premier orifice en position fermée du clapet ;
la base circulaire comprenant un deuxième orifice (412) disposé en face de la base mobile de manière à être obstruable par celle-ci formant ainsi le clapet.

4. Diffuseur selon la revendication 3, dans lequel la base circulaire est prolongée à sa périphérie d'une jupe (414) s'étendant substantiellement perpendiculairement à la base circulaire, la jupe et la base circulaire formant logement pour la pièce mobile et le rappel.

5. Diffuseur selon la revendication 3 ou la revendication 4, dans lequel le rappel est un ressort disposé de manière à forcer la base mobile contre la base circulaire obstruant ainsi le deuxième orifice.

6. Diffuseur selon la revendication 5, comprenant en outre un logement de ressort (43) disposé sous la pièce mobile.

7. Diffuseur selon la revendication 6, dans lequel le logement de ressort est formé d'au moins trois bras coplanaires (432) s'étendant à partir d'un centre (431) et d'autant de doigts de fixation (433), chacun des doigts de fixation prolongeant l'un des trois bras coplanaires perpendiculairement au plan commun des bras coplanaires ;
dans lequel la jupe est pourvue d'autant d'orifices de fixation (415) qu'il y a de doigts de fixation et recevant chacun un des doigts de fixation pour la fixation du logement de ressort à la jupe.

8. Dispositif selon l'une des revendications 1 à 7, comprenant en outre un connecteur à une source d'alimentation.

9. Dispositif selon la revendication 8, dans lequel le connecteur à une source d'alimentation est une fiche mâle ou un connecteur USB.

10. Dispositif selon l'une des revendications 1 à 7, comprenant en outre un module photovoltaïque pour l'alimentation électrique du dispositif.

11. Dispositif selon l'une des revendications 1 à 10, dans lequel le disperseur est un nébuliseur ou une pastille piézoélectrique.

## Patentansprüche

1. Elektrischer Diffusor (10) für ätherisches Öl, umfassend:
- ein Gehäuse (1);
- eine Aufnahme (2) für eine Flasche mit ätherischem Öl, die am Gehäuse vorgesehen und mit einem Befestigungsring (21) versehen ist;
- einen Behälter (5), der mit einem Zerstäuber (6) für ätherisches Öl versehen ist;
**dadurch gekennzeichnet, dass** der Diffusor ferner einen Tropfenabschneider (4) in oder direkt unter der Aufnahme umfasst, der eine geschlossene und eine offene Stellung aufweist;
wobei der Befestigungsring und der Tropfenabschneider derart angeordnet sind, dass der Tropfenabschneider:
- in seiner offenen Stellung ist, wenn ein Fläschchen in der Aufnahme aufgenommen und am Befestigungsring befestigt ist, der das Aufnahmegehäuse und den Behälter in Flüssigkeitskommunikation verbindet; und
- andernfalls in seiner geschlossenen Stellung ist;
wobei der Befestigungsring ein Innengewinde zur Schraubbefestigung des Halses des Fläschchens mit ätherischem Öl aufweist;
wobei der Tropfenabschneider unter dem Befestigungsring aufgenommen ist.

2. Diffusor nach Anspruch 1, wobei der Tropfenabschneider umfasst:
- ein Ventil (412, 421), das zwei Stellungen aufweist, eine geschlossene Stellung, die der geschlossenen Stellung des Tropfenabschneiders entspricht, und eine offene Stellung, die der offenen Stellung des Tropfenabschneiders entspricht;
- einen Aktuator (422) zum Bewegen des Ventils von der geschlossenen Stellung in die offene Stellung;
- eine Rückstellvorrichtung (44) zum Bewegen des Ventils von der offenen in die geschlossene Stellung;
wobei der Befestigungsring und der Tropfenabschneider derart ausgebildet sind, dass beim Befestigen des Flaschenhalses am Befestigungsring letzterer den Aktuator dazu zwingt, das Ventil aus der geschlossenen in die offene Stellung zu bewegen, und dass beim Entfernen des Flaschenhalses vom Befestigungsring die Rückstellvorrichtung das Ventil in seine geschlossene Stellung zurückbringt.

3. Diffusor nach Anspruch 2, wobei der Tropfenabschneider gebildet ist aus:
- einem beweglichen Teil (42), das aus einer beweglichen Basis (421) gebildet ist, von der sich ein oder mehrere Betätigungsfinger (422), die den Aktuator bilden, im Wesentlichen senkrecht zu dieser Basis erstrecken;
- einer kreisförmigen Basis (41), die eine oder mehrere erste Öffnungen (411) umfasst, wobei die Anzahl der ersten Öffnungen gleich der Anzahl der Betätigungsfinger ist und jeder Finger in der geschlossenen Stellung des Ventils durch eine erste Öffnung hindurchführt;
wobei die kreisförmige Basis eine zweite Öffnung (412) aufweist, die gegenüber der beweglichen Basis derart angeordnet ist, dass sie von dieser blockiert werden kann, wodurch das Ventil gebildet wird.

4. Diffusor nach Anspruch 3, wobei die kreisförmige Basis an ihrem Umfang durch eine Schürze (414) verlängert ist, die sich im Wesentlichen senkrecht zur kreisförmigen Basis erstreckt, wobei die Schürze und die kreisförmige Basis eine Aufnahme für das bewegliche Teil und die Rückstellvorrichtung bilden.

5. Diffusor nach Anspruch 3 oder 4, wobei die Rückstellvorrichtung eine Feder ist, die derart angeordnet ist, dass sie die bewegliche Basis gegen die kreisförmige Basis drückt und dadurch die zweite Öffnung verschließt.

6. Diffusor nach Anspruch 5, ferner umfassend ein Federgehäuse (43), das unterhalb des beweglichen Teils angeordnet ist.

7. Diffusor nach Anspruch 6, wobei das Federgehäuse aus wenigstens drei koplanaren Armen (432), die sich von einem Zentrum (431) aus erstrecken, und ebenso vielen Befestigungsfingern (433) gebildet ist, wobei jeder der Befestigungsfinger einen der drei koplanaren Arme senkrecht zur gemeinsamen Ebene der koplanaren Arme verlängert; wobei die Schürze mit so vielen Befestigungslöchern (415) versehen ist, wie es Befestigungsfinger gibt, und jeweils einen der Befestigungsfinger zur Befestigung des Federgehäuses an der Schürze aufnimmt.

8. Vorrichtung nach einem der Ansprüche 1 bis 7, ferner umfassend einen Anschluss an eine Stromquelle.

9. Vorrichtung nach Anspruch 8, wobei der Anschluss an eine Stromquelle ein männlicher Stecker oder ein USB-Anschluss ist.

10. Vorrichtung nach einem der Ansprüche 1 bis 7, ferner umfassend ein Photovoltaikmodul zur elektrischen Versorgung der Vorrichtung.

11. Vorrichtung nach einem der Ansprüche 1 bis 10, wobei der Zerstäuber ein Vernebler oder ein piezoelektrisches Plättchen ist.

## Claims

1. Electric essential oil diffuser (10) comprising:
- a casing (1);
- an accommodating housing (2), for accommodating an essential oil bottle, provided on the casing and equipped with a fixing ring (21);
- a tank (5) provided with an essential oil disperser (6);
**characterized in that** the diffuser further comprises a drip-stopper (4) in the accommodating housing or just below the latter and having a closed position and an open position;
the fixing ring and the drip-stopper being arranged in such a manner that the drip-stopper is:
- in its open position when a bottle is accommodated in the accommodating housing and fixed to the fixing ring fluidly connecting the accommodating housing and the tank; and
- in its closed position otherwise;
wherein the fixing ring has an internal thread for screw fixing the neck of the essential oil bottle;
wherein the drip-stopper is accommodated under the fixing ring.

2. Diffuser according to claim 1, wherein the drip-stopper comprises:
- a valve (412, 421) having two positions, a closed position corresponding to the closed position of the drip-stopper and an open position corresponding to the open position of the drip-stopper;
- an actuator (422) for moving the valve from the closed position to the open position;
- a return member (44) for moving the valve from the open position to the closed position;
the fixing ring and drip-stopper being configured in such a manner that upon fixing the neck of the bottle to the fixing ring, the neck forces the actuator to move the valve from the closed position to the open position, and that when removing the neck of the bottle from the fixing ring, the return member brings the valve back to its closed position.

3. Diffuser according to claim 2, wherein the drip-stopper is made up of:
- a movable part (42) made up of a movable base (421) from which one or more actuating finger(s) (422) forming the actuator extend(s) substantially perpendicular thereto;
- a circular base (41) comprising one or more first opening(s) (411), the number of first openings being equal to the number of actuating fingers, each finger passing through a first opening in the closed position of the valve;
the circular base comprising a second opening (412) arranged opposite the movable base so as to be obstructable by the latter, thereby forming the valve.

4. Diffuser according to claim 3, wherein a skirt (414) extending substantially perpendicular to the circular base project from the periphery of the circular base, wherein the skirt and the circular base form a housing for the moving part and the return member.

5. Diffuser according to claim 3 or claim 4, wherein the return member is a spring arranged in such a manner as to force the mobile base against the circular base thus obstructing the second orifice.

6. Diffuser according to claim 5, further comprising a spring housing (43) arranged under the movable part.

7. Diffuser according to claim 6, wherein the spring housing is made up of at least three coplanar arms (432) extending from a center (431) and of an equal number of fixing fingers (433), each of the fixing fingers projecting from one of the three coplanar arms perpendicular to the common plane of the coplanar arms;
wherein the skirt is provided with as many fixing holes (415) as there are fixing fingers and each accommodating one of the fixing fingers for fixing the spring housing to the skirt.

8. Device according to one of claims 1 to 7, further comprising a connector for connecting to a power source.

9. Device according to claim 8, wherein the connector for connecting to a power source is a male plug or a USB connector.

10. Device according to one of claims 1 to 7, further comprising a photovoltaic module to supply electricity to the device.

11. Device according to one of claims 1 to 10, wherein the disperser is a nebulizer or a piezoelectric pad.
